(19) Europäisches Patentamt
European Patent Office
Office européen des brevets

(11) Veröffentlichungsnummer **0 004 902**
**B1**

(12) # EUROPÄISCHE PATENTSCHRIFT

(45) Veröffentlichungstag der Patentschrift
21.04.82

(51) Int Cl.³ **C 07 D 317/62,** C 07 D 317/68,
A 01 N 43/30

(21) Anmeldenummer 79101016.8

(22) Anmeldetag 04.04.79

(54) **Benzodioxolderivate, Verfahren zu ihrer Herstellung und ihre Verwendung als Synergisten in Schädlingsbekämpfungsmitteln.**

(30) Priorität 14.04.78 DE 2816190

(43) Veröffentlichungstag der Anmeldung
31.10.79 Patentblatt 79/22

(45) Bekanntmachung des Hinweises auf die Patenterteilung
21.04.82 Patentblatt 82/16

(84) Benannte Vertragsstaaten:
BE CH DE FR GB IT NL SE

(56) Entgegenhaltungen
FR-A-2 315 850

(73) Patentinhaber: **BAYER AG, Zentralbereich Patente, Marken und Lizenzen, D-5090 Leverkusen 1, Bayerwerk (DE)**

(72) Erfinder: **Mues, Volker, Dr., Mareèstrasse 61, D-5600 Wuppertal 1 (DE)**
Erfinder **Behrenz, Wolfgang, Dr., Untergruendemich 14, D-5063 Overath (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 902 B1

BUNDESDRUCKEREI BERLIN

Benzodioxolderivate, Verfahren zu ihrer Herstellung und ihre Verwendung als Synergisten in Schädlingsbekämpfungsmitteln

Die vorliegende Erfindung betrifft neue Benzodioxolderivate, mehrere Verfahren zu ihrer Herstellung sowie ihre Verwendung als Synergisten.

Es ist bereits bekannt, daß die folgenden Wirkstoffe bzw. Wirkstoffgruppen pestizide, insbesondere insektizide und akarizide Eigenschaften besitzen:

A) Carbamate, wie z. B. das 2-iso-Propoxy-phenyl-N-methyl-carbamat, 3,4,5-Trimethyl-phenyl-N-methyl-carbamat, 1-Naphthyl-N-methyl-carbamat, 2,3-Dihydro-2,2-dimethyl-7-benzofuranyl-N-methyl-carbamat, 2-(1,3-Dioxolan(2)yl-phenyl-N-methyl-carbamat und 2,2-Dimethyl-1,3-benzodioxol(4)yl-N-methyl-carbamat,

B) Carbonsäureester, wie z. B. 2,3,4,5-Tetrahydrophthalimido-methyl-chrysanthemat und (5-Benzyl-3-furyl)-methyl-2,2-dimethyl-3-(2-methyl-propenyl)-cyclopropan-carboxylat,

C) Phosphorsäureester, wie z. B. O,O-Dimethyl-O-(2,2-dichlorvinyl)-phosphorsäureester und

D) Halogenalkane, wie z. B. 1,1,1-Trichlor-2,2-bis-(4-methoxyphenyl)-äthan und 1,1,1-Trichlor-2,2-bis-(4-chlorphenyl)-äthan.

Weiterhin sind synergistische Mischungen von Carbamaten, z. B. 2-iso-Propoxy-phenyl-N-methyl-carbamat oder von Phosphorsäureestern, z. B. O,O-Diäthyl-O-(2-isopropyl-4-methylpyrimidin(6)yl)-thionophosphorsäureester oder von natürlichen oder synthetischen Pyrethroiden mit Piperonyläthern, wie z. B. α-(2-(2-Butoxy-äthoxy)-äthoxy)-4,5-methylendioxy-2-propyl-toluol, bekannt (vergleiche Bull. Org. mond. Santé/Bull. Wld. Hlth Org. 1966, 35, 691–708; Schrader, G.: Die Entwicklung neuer insektizider Phosphorsäureester 1963, S. 158). Doch ist die Wirksamkeit dieser synergistischen Wirkstoffkombinationen nicht befriedigend. Eine gewisse praktische Bedeutung hat bisher nur das α-(2-(2-Butoxy-äthoxy)-äthoxy)-4,5-methylendioxy-2-propyl-toluol erlangt. Aus der FR-A-2 315 850 ist es ferner bekannt, daß bestimmte Benzodioxol-Derivate eine synergistische Wirkung bei bestimmten herbizid wirkenden N,N-disubstituierten Alanin-Derivaten aufweisen.

Es wurden nun die neuen Benzodioxol-Derivate der Formel (I) gefunden,

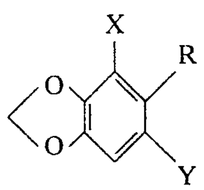

(I)

in welcher

R für Wasserstoff, Halogen, Methyl, Chlormethyl, Formyl, Acetoxymethyl und Cyano sowie für den Rest der Formel

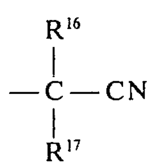

(VI)

worin

$R^{16}$ für Wasserstoff oder Methyl steht,

$R^{17}$ für Wasserstoff, geradkettiges oder verzweigtes Alkyl mit 1 bis 5 Kohlenstoffatomen, Allyl, Propargyl oder Benzyl steht sowie

X und Y für gleiches oder verschiedenes Halogen stehen.

Die neuen Verbindungen der Formel (I) können hergestellt werden, indem man

a) 1,2-Dihydroxybenzole der Formel (VII)

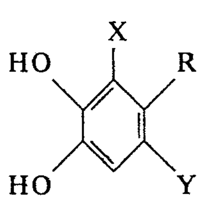

(VII)

worin

R, X und Y die oben angegebene Bedeutung haben,

mit Dihalogenmethanen der Formel (VIII)

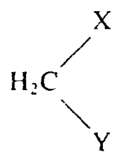

(VIII)

worin

X und Y die oben angegebene Bedeutung haben,

in Gegenwart eines Säureakzeptors und gegebenenfalls in Gegenwart eines Verdünnungsmittels umsetzt, oder indem man

b) Benzodioxolderivate der Formel (I a)

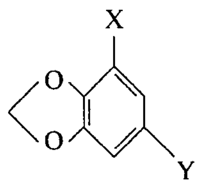

(I a)

in welcher

X und Y die oben angegebene Bedeutung haben

mit Halogenierungsmitteln oder mit Formaldehyd/HCl zu Verbindungen der Formel (I) umsetzt, in der X und Y die oben angegebene Bedeutung haben und R für Halogen oder $CH_2Cl$ steht, oder indem man

c) Benzoldioxolderivate der Formel (I c)

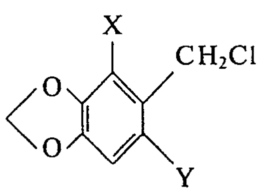

(Ic)

in welcher

X und Y die oben angegebene Bedeutung haben

durch katalytische Hydrierung in Verbindungen der Formel (I) umwandelt, in der R für Methyl steht bzw. durch Reaktion mit Verbindungen, welche das Acetat- bzw. Cyanidion enthalten, z. B. mit Natriumacetat bzw. -cyanid, in Verbindungen der Formel (I) umwandelt, in der R für Acetoxymethyl bzw. Cyanomethyl steht bzw. durch Umsetzung mit Hexamethylentetramin in Verbindungen der Formel (I) überführt, in der R für Formyl steht,

d) Benzodioxolderivate der Formel (I g)

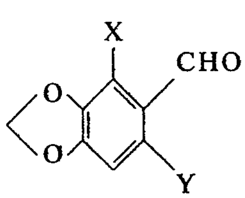

(Ig)

in welcher

X und Y die oben angegebene Bedeutung haben, mit Hydroxylamin in die entsprechenden Oxime überführt und daraus mit Dehydratisierungsmitteln die Verbindungen der Formel (I), in welcher R für Cyano steht, erzeugt,

e) Benzodioxolderivate der Formel (If)

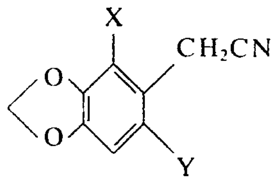

(If)

in welcher

X und Y die oben angegebene Bedeutung haben

mit Halogenalkanen, -alkenen oder -alkinen zu Verbindungen der Formel (II)

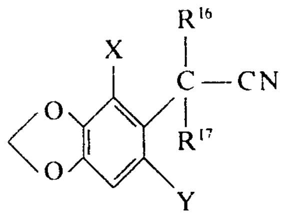

(II)

in welcher

$R^{16}$ für Wasserstoff oder Methyl steht,
$R^{17}$ für Wasserstoff, geradkettiges oder verzweigtes Alkyl mit 1 bis 5, insbesondere 1 bis 3 Kohlenstoffatomen, Allyl, Propargyl oder Benzyl steht und
X und Y die oben angegebene Bedeutung haben, umsetzt.

Die neuen Benzodioxolderivate der Formel I zeigen in Kombination mit

A) Carbamaten und/oder
B) Carbonsäureestern, einschließlich der natürlichen sowie synthetischen Pyrethroide, und/oder
C) Phosphorsäureestern und/oder
D) Halogenalkanen

eine besonders hohe insektizide und akarizide Wirkung.
Die synergistische Wirkung der Verbindungen der allgemeinen Formel I zeigt sich bevorzugt bei

A) Carbamaten der allgemeinen Formel (II)

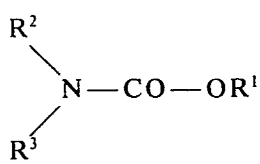

(II)

in welcher

$R^1$ für Aryl, einen Heterocyclus oder einen Oximrest steht,
$R^2$ für Wasserstoff oder einen Alkylrest mit 1 bis 4 Kohlenstoffatomen steht und
$R^3$ für Alkyl, Alkylcarbonyl mit 1 bis 6 Kohlenstoffatomen im Alkylrest, der gegebenenfalls auch durch Hydroxy oder Methylthio substituiert sein kann, oder den Rest $-S-Z$ steht, wobei
Z für einen gegebenenfalls durch Halogen substituierten aliphatischen Rest mit 1 bis 4 Kohlenstoffatomen, insbesondere $CCl_3$ und $CF_3$ sowie für gegebenenfalls bevorzugt durch Nitril, Halogen, insbesondere Chlor, Methyl, Trihalogenmethyl, Trifluormethylmercapto oder Nitro substituierten Arylrest, insbesondere Phenyl, oder für Methoxycarbonyl oder für den Rest

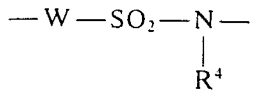

steht, wobei

W für Alkyl, Halogenalkyl oder Alkylamino, Dialkylamino oder einen gegebenenfalls bevorzugt durch Halogen, Trihalogenmethyl, Nitril, Methyl oder Nitro substituierten Arylrest steht.

Besonders bevorzugt sind Carbamate der Formel II, worin

$R^1$ für Phenyl oder Naphthyl steht, die gegebenenfalls substituiert sind durch Alkyl, Alkenyl, Alkoxy, Alkylmercapto oder Alkylthioalkyl mit jeweils 1 bis 6 Kohlenstoffatomen, Dialkylamino, Dialkenylamino mit bis zu 3 Kohlenstoffatomen je Alkyl- bzw. Alkenylteil, Halogen, insbesondere Chlor, Dioxolanyl oder den Rest $-N=CH-N(C_{1-4}\text{-Alkyl})_2$ steht.

Weiterhin sind besonders bevorzugt Carbamate der Formel II, worin

$R^1$ für 2,3-Dihydrobenzofuranyl, Benzodioxol, Benzothienyl, Pyrimidinyl oder Pyrazolyl steht, die gegebenenfalls durch Alkylgruppen mit 1 bis 4 Kohlenstoffatomen, insbesondere Methyl, oder durch Dialkylaminogruppen mit 1 bis 4 Kohlenstoffatomen je Alkylteil substituiert sind.

Weiterhin sind besonders bevorzugt Carbamate der allgemeinen Formel II, worin $R^1$ für einen Rest der Formel (IIa)

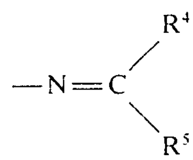

(II a)

steht, in welcher

$R^4$ und $R^5$ gleich oder verschieden sind und für Alkyl, Cycloalkyl, Alkenyl, Alkinyl, Alkoxy, Alkylmercapto, Alkoxycarbonyl, Carbonylamid, Alkylmercaptoalkyl, mit jeweils bis zu 5 Kohlenstoffatomen, Nitril, Aryl, insbesondere Phenyl, einen gegebenenfalls substituierten heterocyclischen Rest oder für Alkyl, das durch einen heterocyclischen Rest substituiert ist oder gemeinsam einen gegebenenfalls durch $C_{1-4}$-Alkyl substituierten Dioxolanyl- oder Dithiolanylrest stehen;

besonders erwähnt seinen folgende Carbamate der Formel II:

2-Methylphenyl-, 2-Äthylenphenyl-, 2-n-Propylphenyl-, 2-Methylphenyl-, 2-Äthoxyphenyl-, 2-iso-Propoxyphenyl-, 4-Methylphenyl-, 4-Äthylphenyl-, 4-n-Propylphenyl-, 4-Methoxyphenyl-, 4-Äthoxyphenyl-, 4-n-Propoxyphenyl-, 3,4,5-Trimethylphenyl-, 1-Naphthyl-, 2,3-Dihydro-2,2-dimethyl-7-benzofuranyl-, 2-(1,3-Dioxolan(2)yl-phenyl)-, bzw. 2,2-Dimethyl-1,3-benzodioxol(4)yl-N-methyl-carbamat und die entsprechenden -N-methyl-N-acetyl-, -N-methyl-N-trifluormethylthio-, -N-methyl-N-dichlormonofluormethylthio- bzw. -N-methyl-N-dimethylaminothio-carbamate.

Weiter zeigt sich die synergistische Wirkung der Verbindungen der allgemeinen Formel I bevorzugt bei

B) Carbonsäureestern der allgemeinen Formel (III)

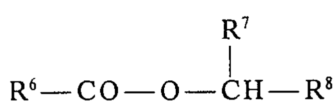

(III)

in welcher

$R^6$ für Alkyl, Aralkyl, Aryl oder Cycloalkyl steht, die gegebenenfalls substituiert sein können,
$R^7$ für Wasserstoff, Alkyl, Halogenalkyl, Alkenyl, Alkinyl, Nitril steht und
$R^8$ für Aryl oder einen Heterocyclus steht, oder gemeinsam mit $R^7$ einen gegebenenfalls substituierten Cyclopentenonring bildet.

Besonders bevorzugt sind Carbonsäureester, in denen $R^6$ für Alkyl mit 1 bis 6 Kohlenstoffatomen, das gegebenenfalls durch gegebenenfalls halogensubstituiertes Phenyl substituiert ist, Cyclopropyl, das gegebenenfalls durch Alkyl, Alkenyl, Halogenalkyl oder Halogenalkenyl mit jeweils bis zu 6 Kohlenstoffatomen substituiert ist, oder für Phenyl, das gegebenenfalls durch Halogen substituiert ist, steht. Bevorzugt sind Carbonsäureester, in denen $R^7$ für Wasserstoff, Alkyl mit 1 bis 6 Kohlenstoffatomen, Halogenalkyl mit 1 bis 4 Kohlenstoffatomen und bis zu 3 Halogenatomen, Nitril oder Äthinyl steht.

Weiter sind besonders bevorzugt Carbonsäureester, in denen $R^8$ für Phenyl steht, das gegebenenfalls durch $C_{1-4}$-Alkyl, Halogen, insbesondere Fluor oder Chlor, gegebenenfalls halogen- oder methyl-substituiertes Phenoxy, gegebenenfalls substituiertes Benzyl substituiert ist, ferner für Furanyl, Tetrahydrophthalimido, Benzodioxol, die gegebenenfalls durch Halogen, insbesoncere Chlor, Alkyl oder Alkenyl mit bis zu 4 Kohlenstoffatomen oder Benzyl substituiert sind, steht, und ferner für Cyclopentonon steht, das gegebenenfalls durch $C_{1-4}$-Alkyl, Furfuryl, $C_{1-5}$-Alkenyl substituiert ist.

Im einzelnen seien genannt:

Essigsäure-(1-(3,4-dichlorphenyl)-2,2,2-trichloräthyl)-ester,
2,3,4,5-Tetrahydrophthalimidomethylchrysanthemat und
(5-Benzyl-3-furyl)-methyl-2,2-dimethyl-3-(2-methylpropenyl)-cyclopropancarboxylat.

Weiter sind besonders bevorzugt die natürlich vorkommenden Pyrethroide.
Weiter zeigt sich die synergistische Wirkung der Verbindungen der allgemeinen Formel I bevorzugt bei

C) Phosphorsäureestern der allgemeinen Formel IV

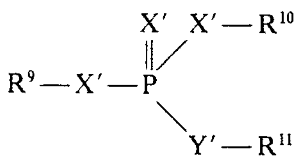

(IV)

in welcher

| | |
|---|---|
| X' | unabhängig voneinander für O oder S steht und |
| Y' | für O, S, –NH– oder für eine direkte Bindung zwischen dem zentralen P-Atom und dem $R^{11}$ steht und |
| $R^9$ und $R^{10}$ | gleich oder verschieden sind und für Alkyl oder Aryl stehen, |
| $R^{11}$ | für Alkyl, Aryl, Heteroaryl, Aralkyl, Alkenyl, Dioxanyl, oder einen Oximrest oder für den gleichen Rest steht, an den es gebunden ist. |

Besonders bevorzugt sind Phosphorsäureester, in denen

$R^9$ und $R^{10}$ gleich oder verschieden sind und für $C_{1-4}$-Alkyl oder Phenyl stehen,

$R^{11}$ für Alkyl mit 1 bis 4 Kohlenstoffatomen steht, das gegebenenfalls durch Halogen, Hydroxyl, Nitril, gegebenenfalls halogensubstituiertes Phenyl, Carbonylamid, Sulfonylalkyl, Sulfoxyalkyl, Alkylcarbonyl, Alkoxy, Alkylmercapto, Alkoxycarbonyl, substituiert ist, für Alkenyl mit bis zu 4 Kohlenstoffatomen, das gegebenenfalls durch Halogen, gegebenenfalls halogensubstituiertes Phenyl oder Alkoxycarbonyl substituiert ist, oder für den Oximrest der allgemeinen Formel

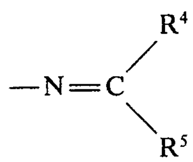

(IIa)

wobei $R^4$ und $R^5$ die oben angegebene Bedeutung besitzen, insbesondere jedoch für Cyan oder Phenyl stehen,

$R^{11}$ steht ferner für Dioxanyl, das durch denselben Rest substituiert ist, an den $R^{11}$ gebunden ist, oder $R^{11}$ steht für den gleichen Rest, an den er gebunden ist, oder $R^{11}$ steht für Phenyl, das gegebenenfalls durch Methyl, Nitro, Nitril, Halogen, Methylthio substituiert ist; $R^{11}$ steht außerdem besonders bevorzugt für gegebenenfalls durch $C_{1-4}$-Alkyl, Halogen substituierte Heteroaromaten, wie Pyridin, Chinolin, Chinoxalin, Pyrimidin, Diazinon, Benzo-1,2,4-triazin;

im einzelnen seien genannt:

O,O-Dimethyl- bzw. O,O-Diäthyl-O-(2,2-dichlor- bzw. 2,2-dibromvinyl)-phosphorsäureester,
O,O-Diäthyl-O-(4-nitro-phenyl)-thionophosphorsäureester,
O,O-Dimethyl-O-(3-methyl-4-methylthio)-thionophosphorsäureester,
O,O-Dimethyl-O-(3-methyl-4-nitro)-thionophosphorsäureester,
O-Äthyl-S-n-propyl-O-(2,4-dichlorphenyl)-thionothiolphosphorsäureester,
O-Äthyl-S-n-propyl-O-(4-methylthio-phenyl)-thionothiolphosphorsäureester,
O,O-Dimethyl-S-(4-oxo-1,2,3-benzotriazin(3)yl-methyl)-thionothiol-phosphorsäureester,
O-Methyl-O-(2-iso-propyl-6-methoxy-pyrimidin(4)yl)-thionomethan-phosphonsäureester,
O,O-Diäthyl-O-(2-iso-propyl-6-methyl-pyrimidin(4)yl)-thionophosphorsäureester,
O,O-Diäthyl-O-(3-chlor-4-methyl-cumarin(7)yl)-thionophosphorsäureester,
O,O-Dimethyl-2,2,2-trichlor-1-hydroxy-äthan-phosphonsäureester,
O,O-Dimethyl-S-(methylcarbamoylmethyl)-thionophosphorsäureester.

Weiter zeigt sich die synergistische Wirkung der Verbindungen der allgemeinen Formel I bevorzugt bei

D) Halogenalkanen der Formel

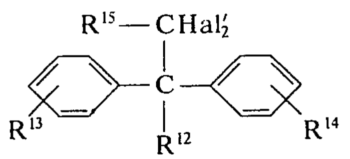

(V)

in welcher

Hal' für Chlor oder Brom und
$R^{12}$ für Wasserstoff oder Hydroxyl stehen,
$R^{13}$ und $R^{14}$ gleich oder verschieden sind und für Halogen, Alkyl oder Alkoxy und
$R^{15}$ für Wasserstoff oder Halogen stehen.

Besonders bevorzugt sind Halogenalkane, in denen

$R^{12}$ Wasserstoff oder Hydroxyl bedeutet,
$R^{13}$ und $R^{14}$ für gleiches Halogen, Alkyl bzw. Alkoxy mit 1 bis 4 Kohlenstoffatomen je Alkyl- bzw. Alkoxyrest stehen und
$R^{15}$ Halogen bedeutet.

Im einzelnen seien genannt:

1,1,1-Trichlor-2,2-bis(4-chlor- bzw. 4-methoxyphenyl)-äthan,
1,1,1-Trichlor-2-hydroxy-2,2-bis(4-chlorphenyl)-äthan und
1,1-Dichlor-2,2-bis(4-äthylphenyl)-äthan.

Überraschenderweise ist die insektizide und/oder akarizide Wirkung der erfindungsgemäßen Wirkstoffkombinationen wesentlich höher als die Wirkung der Einzelkomponenten bzw. die Summe der Wirkungen der Einzelkomponenten. Sie ist ferner wesentlich höher als die Wirkung der bereits bekannten Wirkstoffkombination aus 2-iso-Propoxy-phenyl-N-methyl-carbamat und Piperonylbutoxyd. Außerdem zeigen die erfindungsgemäß verwendbaren Benzodioxol-Derivate ausgezeichnete synergistische Wirksamkeit nicht nur bei einer Wirkstoffklasse, sondern bei Wirkstoffen aus den verschiedensten chemischen Stoffgruppen.

Somit stellen die erfindungsgemäßen Benzodioxol-Derivate und die sie enthaltenden synergistischen Mischungen eine wertvolle Bereicherung der Technik dar.

Die für die erfindungsgemäße Kombination zu verwendenden Synergisten sind durch die Formel I allgemein definiert. Vorzugsweise stehen darin jedoch

R für Wasserstoff, Chlor, Brom, Methyl, Chlormethyl, Formyl, Acetoxymethyl und Cyano sowie für den Rest der Formel

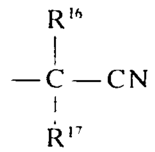 (VI)

worin

$R^{16}$ für Wasserstoff oder Methyl steht,
$R^{17}$ für Wasserstoff, geradkettiges oder verzweigtes Alkyl mit 1 bis 5, insbesondere 1 bis 3 Kohlenstoffatomen, Allyl, Propargyl oder Benzyl steht sowie
X und Y für Fluor, Chlor, Brom oder Jod stehen.

Als Beispiele für die erfindungsgemäßen Benzodioxole der Formel I seien im einzelnen genannt:

3,5-Difluor-, 3,5-Dichlor-, 3,5-Dibrom-, 3,5-Dijod-, 3-Fluor-5-chlor-, 3-Fluor-5-brom-, 3-Fluor-5-jod-, 3-Chlor-5-fluor-, 3-Chlor-5-brom-, 3-Chlor-5-jod-, 3-Brom-5-fluor, 3-Brom-5-chlor-, 3-Brom-5-jod-, 3-Jod-5-fluor-, 3-Jod-5-chlor-, 3-Jod-5-brom-1,2-methylendioxybenzol,
3,5-Difluor-4-brom-, 3,5-Dichlor-4-brom-, 3,4,5-Tribrom-, 3,5-Dijod-4-brom-, 3-Fluor-5-chlor-4-brom-, 3-Fluor-4,5-dibrom-, 3-Fluor-5-jod-4-brom-, 3-Chlor-5-fluor-4-brom-, 3-Chlor-4,5-dibrom-, 3-Chlor-5-jod-4-brom-, 3,4-Dibrom-5-fluor-, 3,4-Dibrom-5-chlor-, 3,4-Dibrom-5-jod-, 3-Jod-5-fluor-4-brom-, 3-Jod-5-chlor-4-brom-, 3-Jod-4,5-dibrom-1,2-methylendioxybenzol,
3,5-Difluor-4-chlor-, 3,4,5-Trichlor-, 3,5-Dibrom-4-chlor-, 3,5-Dijod-4-chlor-, 3-Fluor-4,5-dichlor-, 3-Fluor-5-brom-4-chlor-, 3-Fluor-5-jod-4-chlor-, 3,4-Dichlor-5-fluor-, 3,4-Dichlor-5-brom-, 3,4-Dichlor-5-jod-, 3-Brom-5-fluor-4-chlor-, 3-Brom-4,5-dichlor-, 3-Brom-5-jod-4-chlor-, 3-Jod-5-fluor-4-chlor-, 3-Jod-4,5-dichlor-, 3-Jod-5-brom-4-chlor-1,2-methylendioxybenzol, ferner
3,5-Difluor-, 3,5-Dichlor-, 3,5-Dibrom-, 3,5-Dijod-, 3-Fluor-5-chlor-, 3-Fluor-5-brom-, 3-Fluor-5-jod-, 3-Chlor-5-fluor-, 3-Chlor-5-brom-, 3-Chlor-5-jod-, 3-Brom-5-fluor-, 3-Brom-5-chlor-, 3-Brom-5-jod-, 3-Jod-5-fluor-, 3-Jod-5-chlor-, 3-Jod-5-brom-4-methyl-, -4-chlormethyl-, -4-formyl-, -4-acetoxymethyl-, -4-cyano-, -4-cyanomethyl-, -4-(1-cyano-äthyl)-, -4-(1-cyano-n-propyl)-, -4-(1-cyano-isopropyl)-, -4-(1-cyano-n-butyl)-, -4-(1-cyano-iso-butyl)-, -4-(1-cyano-sek.-butyl)-, -4-(1-cyano-buten(3)yl)-, -4-(1-cyano-butin(3)yl)-, -4-(1-cyano-2-phenyl-äthyl)-1,2-methylendioxybenzol.

Verwendet man 3,4-Dichlor-5-brom-1,2-dihydroxybenzol und Methylenchlorid als Ausgangsstoffe, läßt sich die Herstellungsvariante a) durch folgendes Formelschema wiedergeben:

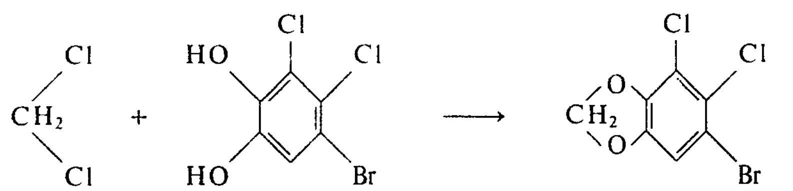

Aus Benzodioxolen der Formel I, in welcher R für Wasserstoff steht (Formel Ia) und welche nach der oben erläuterten Methode hergestellt werden können, lassen sich nach den folgenden Formelschemata erfindungsgemäße Benzodioxolderivate herstellen:
Durch Umsetzung mit Halogenierungsmitteln wie Sulfurylchlorid oder Brom erhält man Verbindungen der Formel Ib, worin Hal für Chlor oder Brom steht. Reaktion mit Formaldehyd/Salzsäure ergibt die Chlormethyl-benzodioxole der Formel Ic.

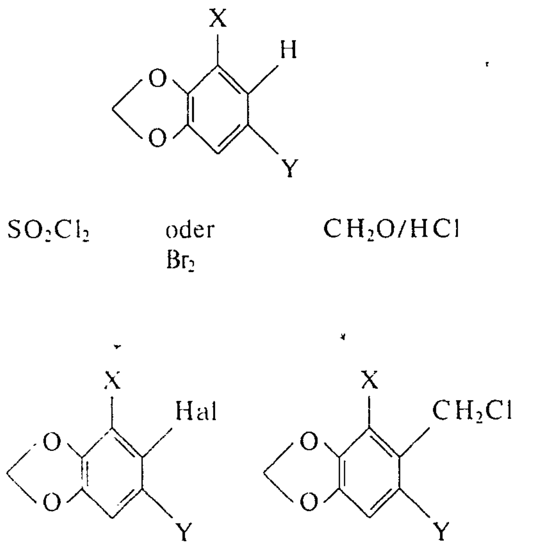


(Ia)

(Ib) (Ic)

Die Chlormethyl-benzodioxole (Ic) können zur Herstellung der weiteren erfindungsgemäßen Wirkstoffe eingesetzt werden. Sie reagieren mit Wasserstoff übergangsmetallkatalysiert unter Bildung von 4-Methyl-benzodioxolen (Id), mit Acetat bilden sie 4-Acetoxymethylbenzodioxole (Ie), mit Cyanid 4-Cyanomethyl-benzodioxole (If).

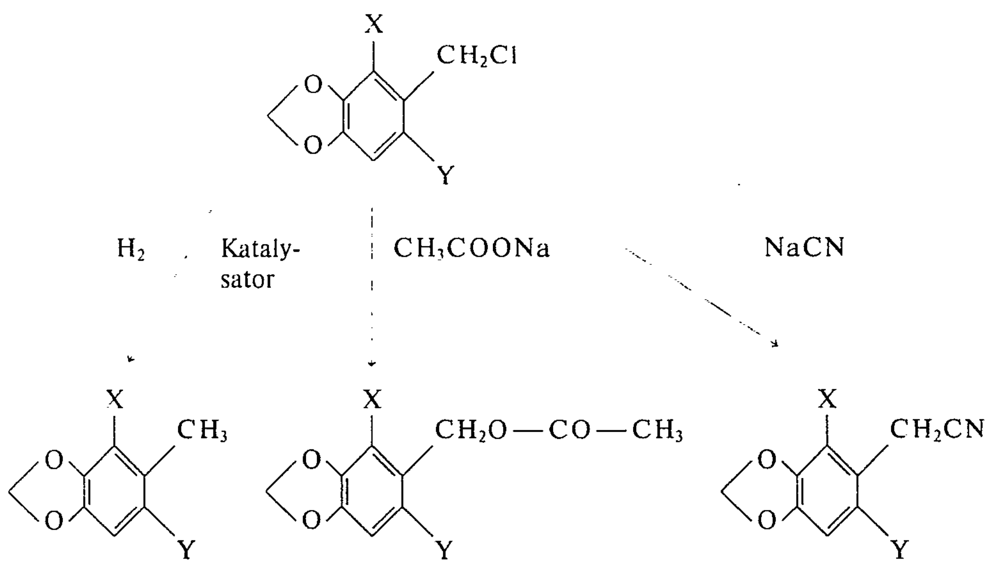


(Ic)

(Id) (Ie) (If)

Umsetzung der Chlormethyl-benzodioxole (Ic) mit Hexamethylentetramin ergibt Formyl-benzodioxole (Ig). Diese können mit Hydroxylamin in Oxime (Ih) und letztere mit Dehydratisierungsmitteln, wie z. B. Acetanhydrid, weiter in Nitrile (Ik) überführt werden:

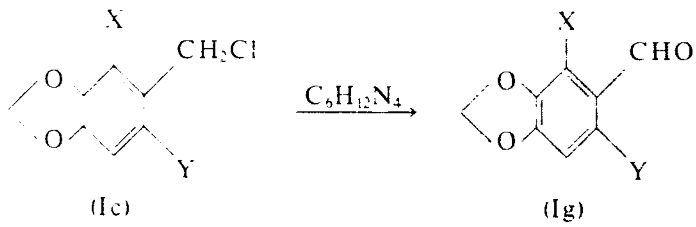


(Ic) (Ig)

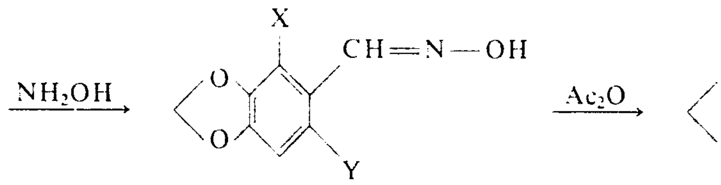


(Ih) (Ik)

Aus den Cyanomethyl-benzodioxolen (If) können durch Umsetzung mit den entsprechenden Halogenalkanen-alkenen oder -alkinen die weiteren erfindungsgemäßen Cyanoalkyl-, Cyanoalkenyl- oder Cyanoalkinyl-benzodioxole hergestellt werden (vergleiche DE-OS 2 215 496; Pure Appl. Chem. 43 (1975), S. 439; Org. Syn. 55 (1976), S. 91):

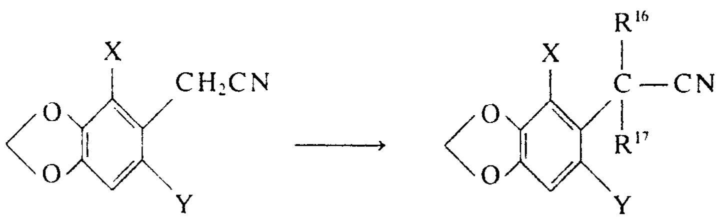

(If) (Il)

Die als Vorprodukte zur Herstellung der erfindungsgemäßen Benzodioxole (I) zu verwendenden 1,2-Dihydroxybenzole (VII) können aus den teilweise bekannten 2-Hydroxy-benzaldehyd-Derivaten, wie z. B. 3,5-Dichlorsalicylaldehyd, durch Reaktion mit Wasserstoffperoxid hergestellt werden nach der sogenannten Dakin-Oxydation (vergleiche Am. Chem. J. 42, 488; GB-PS 794 885; Biochem. Journal 59 [1955], S. 410–415; Chem. Abstr. 49 [1955], 7675h) des folgenden Formelschemas:

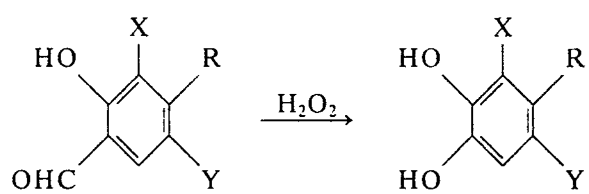


Wie bereits erwähnt, zeigen die neuen Wirkstoffkombinationen der erfindungsgemäßen Benzodioxol-Derivate der Formel (I) mit Carbamaten, Carbonsäureestern, Phosphorsäureestern und/oder Halogenalkanen eine hervorragende Wirkungssteigerung gegenüber den Einzelwirkstoffen bzw. gegenüber deren Summe.

Die Gewichtsverhältnisse der Wirkstoffgruppen können dabei in relativ großen Bereichen schwanken. Im allgemeinen werden die Benzodioxolderivate mit den übrigen Wirkstoffen im Verhältnis 0,1 : 10 bis 10 : 0,1 eingesetzt. Als besonders geeignet haben sich jedoch Mischverhältnisse von 0,5 : 1,0 bis 3,0 : 1,0 erwiesen. Diese Wirkstoffkombinationen bewirken nicht nur eine schnelle knock-down-Wirkung, sondern bewirken auch die nachhaltige Abtötung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normale sensible und resistente Arten sowie gegen alle oder einzelne Entwicklungsstadien wirksam. Zu den obenerwähnten Schädlingen gehören:

Aus der Ordnung der Isopoda z. B. Oniscus asellus, Armadillidium vulgare, Procellio scaber.
Aus der Ordnung der Diplopoda z. B. Blaniulus guttulatus.
Aus der Ordnung der Chilopoda z. B. Geophilus carpophaus, Scutigera spec.
Aus der Ordnung der Symphyla z. B. Scutigerella immaculata.

Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips fermoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffkombinationen können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsions-Konzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol

sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 99 Gew.-% Wirkstoff, vorzugsweise zwischen 0,01 und 10 Gew.-%, liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

$LT_{100}$-Test

Testtiere: Musca domestica, Stamm Weymanns
(gegen Carbamate und Phosphorsäureester resistent)
Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipettiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100%ige knock-down-Wirkung erforderlich ist. Wird die LT nach 6 Stunden nicht erreicht, wird der %-Satz der knock-down gegangenen Testtiere festgestellt.

Konzentrationen der Wirkstoffe, Synergisten und Gemische und ihre Wirkungen gehen aus der nachfolgenden Tabelle hervor.

Tabelle

LT 100-Test mit phosphorsäureester-resistenten Musca domestica (Stamm Weymanns)

| Wirkstoffe bzw. Synergisten ( ) Kennbuchstabe | ( ) Beispiel Nr. | Konzentration in % | LT 100 nach Minuten |
|---|---|---|---|
| 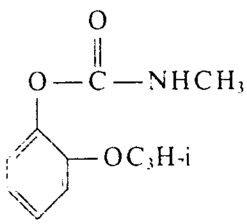 (A) | | 1,0 | 360′ = 0% |
| 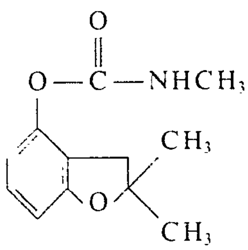 (B) | | 1,0 | 360′ = 0% |
| 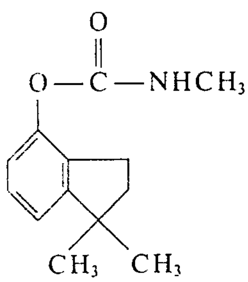 (C) | | 1,0 | 360′ = 0% |
| 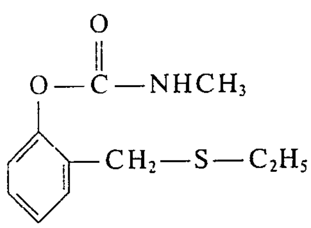 (D) | | 1,0 | 360′ = 0% |
| 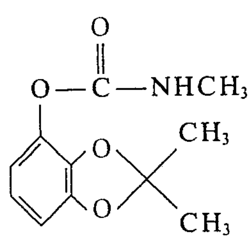 (E) | | 1,0 | 360′ = 0% |

Fortsetzung

| Wirkstoffe bzw. Synergisten<br>( ) Kennbuchstabe ( ) Beispiel Nr. | Konzen-tration in % | LT 100 nach Minuten |
|---|---|---|
| 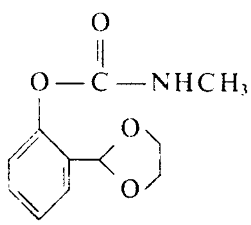<br>(F) | 1,0 | 360′ = 0% |
| 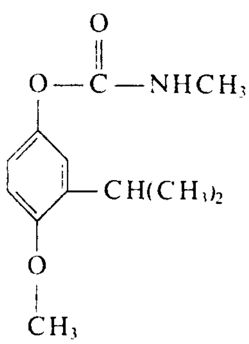<br>(G) | 1,0 | 360′ = 15% |
| 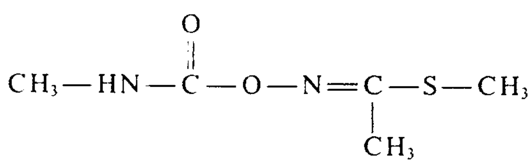<br>(H) | 0,04 | 360′ = 0% |
| Pyrethrine als 25%iger Extrakt<br>(K) | 0,04 | 360′ = 60% |
| 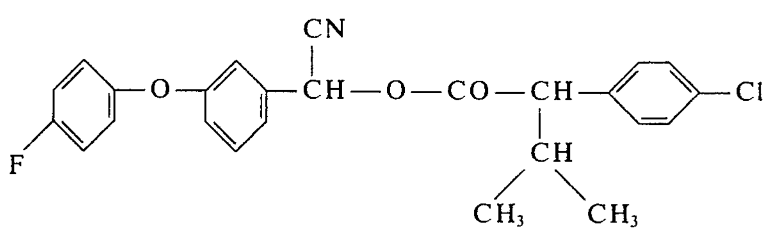<br>(L) | 0,008 | 360′ = 40% |
| 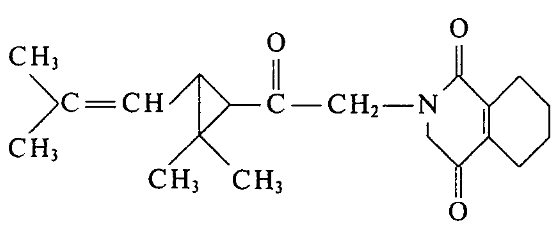<br>(M) | 0,04 | 360′ = 45% |

Fortsetzung

| Wirkstoffe bzw. Synergisten<br>( ) Kennbuchstabe ( ) Beispiel Nr. | Konzentration in % | LT 100 nach Minuten |
|---|---|---|
| 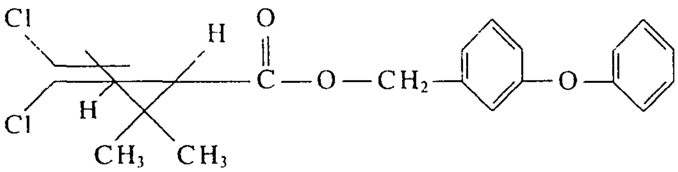<br>(N) | 0,008 | 360′ = 95% |
| 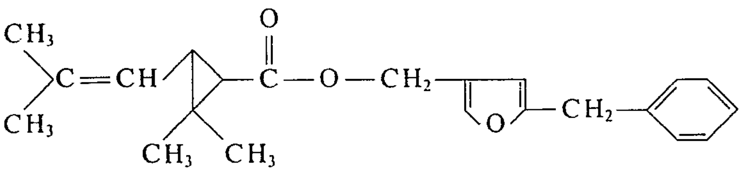<br>(O) | 0,04 | 90′ |
| 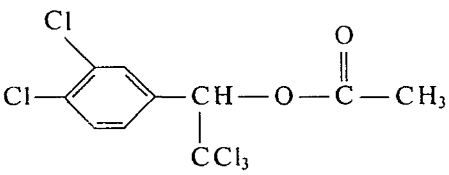<br>(P) | 1,0 | 360′ = 10% |
| 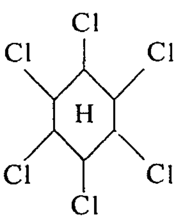<br>(Q) | 1,0 | 360′ |
| 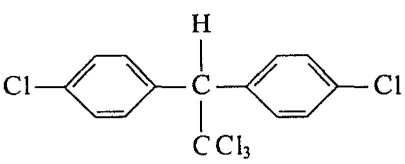<br>(R) | 1,0 | 360′ = 5% |
| 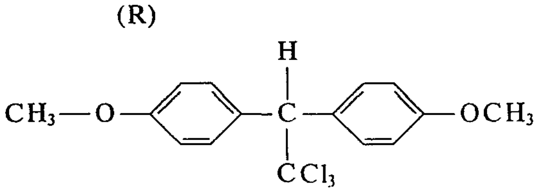<br>(S) | 1,0 | 360′ = 20% |
| 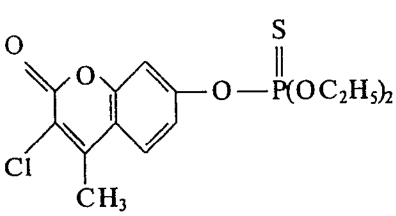<br>(T) | 1,0 | 360′ = 0% |

Fortsetzung

| Wirkstoffe bzw. Synergisten ( ) Kennbuchstabe | ( ) Beispiel Nr. | Konzentration in % | LT 100 nach Minuten |
|---|---|---|---|
| 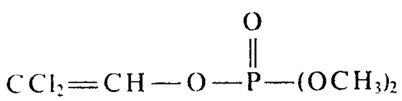 (U) | | 0,008 | 360′ = 90% |
| 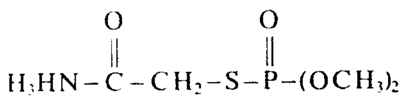 (V) | | 0,04 | 360′ = 25% |
| 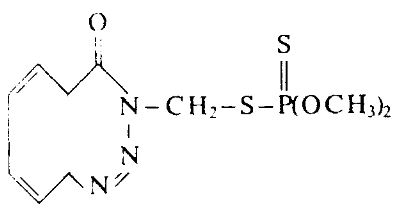 (W) | | 1,0 | 360′ = 65% |
| 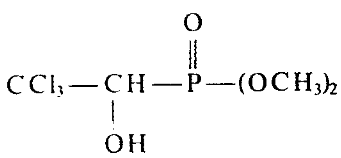 (X) | | 1,0 | 360′ = 45% |
| 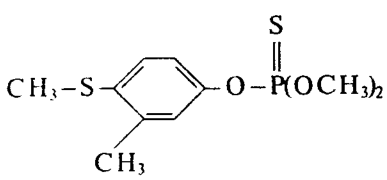 (Y) | | 0,2 | 360′ = 10% |
| 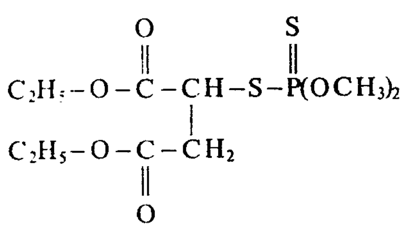 (Z) | | 1,0 | 360′ = 65% |
| | (1) 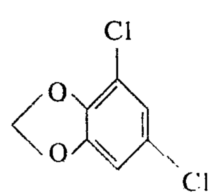 | 0,2 | 360′ = 0% |

Fortsetzung

| Wirkstoffe bzw. Synergisten<br>( ) Kennbuchstabe | ( ) Beispiel Nr. | Konzentration in % | LT 100 nach Minuten |
|---|---|---|---|
| | (12) 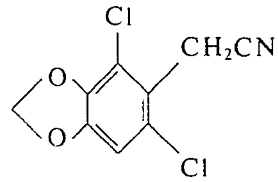 | 1,0 | 360′ = 0% |
| | (13) 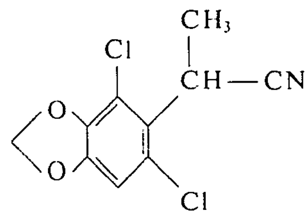 | 1,0 | 360′ = 0% |
| | (14) 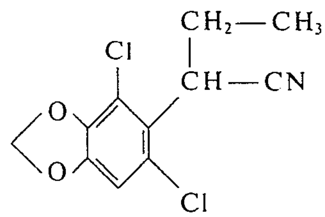 | 1,0 | 360′ = 0% |
| | (15) 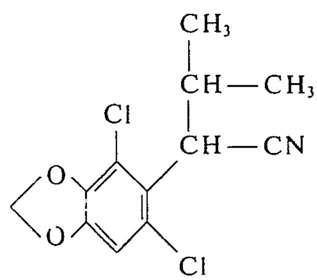 | 1,0 | 360′ = 0% |
| | (10) 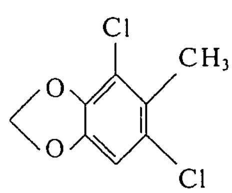 | 1,0 | 360′ = 0% |
| | (2) 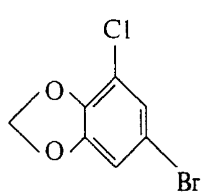 | 0,2 | 360′ = 0% |
| | (6) 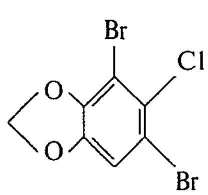 | 1,0 | 360′ = 0% |

Fortsetzung

| Wirkstoffe bzw. Synergisten ( ) Kennbuchstabe | ( ) Beispiel Nr. | Konzentration in % | LT 100 nach Minuten |
|---|---|---|---|
| | (7) 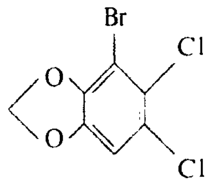 | 1,0 | 360′ = 0% |
| | (17) 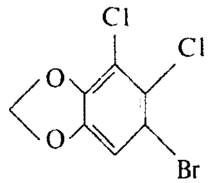 | 1,0 | 360′ = 0% |

| Wirkstoffe bzw. Synergisten ( ) Kennbuchstabe | | ( ) Beispiel-Nr. | Konzentrationen in % | LT 100 nach Minuten |
|---|---|---|---|---|
| | | Vergleichsmittel: | | |
| | | Piperonylbutoxid | 1,0 | 360′ = 0% |
| A | + | Piperonylbutoxid | 0,04 + 0,04 | 360′ = 0% |
| A | + | 1 | 0,008 + 0,008 | 150′ |
| A | + | 12 | 0,04 + 0,04 | 360′ = 95% |
| A | + | 13 | 0,04 + 0,04 | 360′ |
| A | + | 14 | 0,04 + 0,04 | 360′ |
| A | + | 15 | 0,04 + 0,04 | 210′ |
| A | + | 10 | 0,008 + 0,008 | 360′ |
| A | + | 2 | 0,04 + 0,04 | 105′ |
| A | + | 6 | 0,008 + 0,008 | 360′ = 85% |
| A | + | 7 | 0,04 + 0,04 | 180′ |
| A | + | 17 | 0,04 + 0,04 | 360′ = 75% |

Fortsetzung

| Wirkstoffe bzw. Synergisten ( ) Kennbuchstabe | | ( ) Beispiel-Nr. | Konzentrationen in % | LT 100 nach Minuten |
|---|---|---|---|---|
| B | + | Piperonylbutoxid | 0,2 + 0,2 | 360′ = 95% |
| B | + | 1 | 0,008 + 0,008 | 210′ |
| B | + | 13 | 0,04 + 0,04 | 240′ |
| B | + | 14 | 0,2 + 0,2 | 240′ |
| B | + | 15 | 0,04 + 0,04 | 210′ |
| B | + | 10 | 0,008 + 0,008 | 210′ |
| B | + | 2 | 0,008 + 0,008 | 150′ |
| C | + | Piperonylbutoxid | 1,0 + 1,0 | 360′ = 0% |
| C | + | 1 | 1,0 + 1,0 | 150′ |
| C | + | 13 | 1,0 + 1,0 | 360′ = 80% |
| C | + | 14 | 1,0 + 1,0 | 360′ = 95% |
| C | + | 15 | 1,0 + 1,0 | 360′ |
| C | + | 10 | 0,2 + 0,2 | 360′ = 95% |
| C | + | 2 | 0,2 + 0.2 | 360′ |
| D | + | Piperonylbutoxid | 0,2 + 0,2 | 360′ = 0% |
| D | + | 1 | 0,2 + 0,2 | 150′ |
| D | + | 14 | 0,2 + 0,2 | 360′ = 70% |
| D | + | 15 | 0,2 + 0,2 | 360′ = 80% |
| D | + | 10 | 0,2 + 0,2 | 240′ |
| D | + | 2 | 0,2 + 0,2 | 120′ |
| E | + | Piperonylbutoxid | 0,2 + 0,2 | 360′ = 10% |
| E | + | 1 | 0,008 + 0,008 | 360′ = 95% |
| E | + | 12 | 0,04 + 0,04 | 240′ |
| E | + | 13 | 0,04 + 0,04 | 180′ |
| E | + | 14 | 0,04 + 0,04 | 180′ |
| E | + | 15 | 0,04 + 0,04 | 150′ |
| E | + | 10 | 0,008 + 0,008 | 210′ |
| E | + | 2 | 0,008 + 0,008 | 180′ |

Fortsetzung

| Wirkstoffe bzw. Synergisten ( ) Kennbuchstabe | | ( ) Beispiel-Nr | Konzentrationen in % | LT 100 nach Minuten |
|---|---|---|---|---|
| F | + | Piperonylbutoxid | 0,2 + 0,2 | 360' = 5% |
| F | + | 1 | 0,2 + 0,2 | 150' |
| F | + | 12 | 0,2 + 0,2 | 360' = 75% |
| F | + | 13 | 0,04 + 0,04 | 360' = 70% |
| F | + | 10 | 0,04 + 0,04 | 360' = 80% |
| F | + | 2 | 0,04 + 0,04 | 360' = 90% |
| G | + | Piperonylbutoxid | 0,04 + 0,04 | 360' = 15% |
| G | + | 1 | 0,008 + 0,008 | 360' |
| G | + | 10 | 0,008 + 0,008 | 360' = 90% |
| G | + | 2 | 0,04 + 0,04 | 180' |
| H | + | Piperonylbutoxid | 0,2 + 0,2 | 360' = 95% |
| H | + | 1 | 0,04 + 0,04 | 360' |
| H | + | 13 | 0,2 + 0,2 | 180' |
| H | + | 14 | 0,2 + 0,2 | 150' |
| H | + | 15 | 0,04 + 0,04 | 360' |
| H | + | 10 | 0,04 + 0,04 | 150' |
| H | + | 2 | 0,04 + 0,04 | 150' |
| K | + | Piperonylbutoxid | 0,04 + 0,04 | 360' = 95% |
| K | + | 15 | 0,04 + 0,04 | 150' |
| L | + | 1 | 0,008 + 0,008 | 240' |
| L | + | 10 | 0,008 + 0,008 | 240' |
| L | + | 2 | 0,008 + 0,008 | 360' |

Fortsetzung

| Wirkstoffe bzw. Synergisten ( ) Kennbuchstabe | | ( ) Beispiel-Nr. | Konzentrationen in % | LT 100 nach Minuten |
|---|---|---|---|---|
| M | + | Piperonylbutoxid | 0,04 + 0,04 | 360′ |
| M | + | 1 | 0,04 + 0,04 | 150′ |
| M | + | 13 | 0,04 + 0,04 | 150′ |
| M | + | 14 | 0,04 + 0,04 | 150′ |
| M | + | 15 | 0,04 + 0,04 | 150′ |
| M | + | 10 | 0,04 + 0,04 | 60′ |
| M | + | 2 | 0,04 + 0,04 | 60′ |
| N | + | 1 | 0,008 + 0,008 | 120′ |
| N | + | 13 | 0,008 + 0,008 | 150′ |
| N | + | 14 | 0,008 + 0,008 | 150′ |
| N | + | 15 | 0,008 + 0,008 | 210′ |
| N | + | 2 | 0,008 + 0,008 | 105′ |
| O | + | Piperonylbutoxid | 0,04 + 0,04 | 90′ |
| O | + | 1 | 0,04 + 0,04 | 45′ |
| O | + | 10 | 0,04 + 0,04 | 45′ |
| O | + | 2 | 0,04 + 0,04 | 45′ |
| P | + | Piperonylbutoxid | 0,2 + 0,2 | 360′ = 50% |
| P | + | 1 | 0,2 + 0,2 | 150′ |
| P | + | 12 | 0,2 + 0,2 | 240′ |
| P | + | 13 | 0,2 + 0,2 | 240′ |
| P | + | 14 | 0,2 + 0,2 | 180′ |
| P | + | 15 | 0,2 + 0,2 | 210′ |
| P | + | 10 | 0,2 + 0,2 | 360′ = 90% |
| P | + | 2 | 0,2 + 0,2 | 360′ = 95% |
| P | + | 6 | 0,2 + 0,2 | 360′ = 90% |

Fortsetzung

| Wirkstoffe bzw. Synergisten ( ) Kennbuchstabe | | ( ) Beispiel-Nr. | Konzentrationen in % | LT 100 nach Minuten |
|---|---|---|---|---|
| Q | + | Piperonylbutoxid | 0,04 + 0,04 | 360′ = 90% |
| Q | + | 1 | 0,04 + 0,04 | 240′ |
| Q | + | 12 | 0,04 + 0,04 | 210′ |
| Q | + | 13 | 0,04 + 0,04 | 240′ |
| Q | + | 14 | 0,04 + 0,04 | 180′ |
| Q | + | 15 | 0,04 + 0,04 | 180′ |
| R | + | Piperonylbutoxid | 1,0 + 1,0 | 360′ = 20% |
| R | + | 1 | 0,2 + 0,2 | 120′ |
| R | + | 10 | 0,2 + 0,2 | 360′ = 95% |
| R | + | 2 | 0,2 + 0,2 | 180′ |
| S | + | Piperonylbutoxid | 0,2 + 0,2 | 360′ = 70% |
| S | + | 1 | 0,2 + 0,2 | 75′ |
| S | + | 10 | 0,2 + 0,2 | 210′ |
| S | + | 2 | 0,2 + 0,2 | 105′ |
| U | + | Piperonylbutoxid | 0,008 + 0,008 | 210′ |
| U | + | 1 | 0,008 + 0,008 | 120′ |
| U | + | 13 | 0,008 + 0,008 | 75′ |
| U | + | 15 | 0,008 + 0,008 | 180′ |
| U | + | 10 | 0,008 + 0,008 | 120′ |
| V | + | Piperonylbutoxid | 0,04 + 0,04 | 360′ = 60% |
| V | + | 1 | 0,04 + 0,04 | 210′ |
| V | + | 10 | 0,04 + 0,04 | 150′ |
| V | + | 2 | 0,04 + 0,04 | 240′ |
| W | + | Piperonylbutoxid | 0,2 + 0,2 | 360′ = 95% |
| W | + | 1 | 0,2 + 0,2 | 210′ |

Fortsetzung

| Wirkstoffe bzw. Synergisten ( ) Kennbuchstabe | | ( ) Beispiel-Nr. | Konzentrationen in % | | LT 100 nach Minuten |
|---|---|---|---|---|---|
| X | + | Piperonylbutoxid | 1,0 | + 1,0 | 360' = 90% |
| X | + | 1 | 1,0 | + 1,0 | 180' |
| X | + | 10 | 1,0 | + 1,0 | 210' |
| X | + | 2 | 1,0 | + 1,0 | 210' |
| Y | + | Piperonylbutoxid | 0,2 | + 0,2 | 360' = 20% |
| Y | + | 1 | 0,2 | + 0,2 | 360' |
| Z | + | Piperonylbutoxid | 1,0 | + 1,0 | 360' = 45% |
| Z | + | 1 | 0,2 | + 0,2 | 360' |
| Z | + | 2 | 0,2 | + 0,2 | 360' |

## Herstellungsbeispiele

### a) Herstellung von Benzodioxolen der Formel I

Zu einer Lösung von 2,0 Mol Brenzcatechinderivat (VII) in 1500 ml Dimethylformamid gibt man unter Rühren 4,4 Mol Kaliumcarbonat, erwärmt die Suspension auf 60–70°C und tropft 2,2 Mol Chlorbrommethan zu. Die Temperatur steigt dabei an und wird durch Kühlen bei 95°C gehalten. Nach dem Abklingen der Reaktion rührt man noch 5 Stunden bei 100°C, filtriert und destilliert vom Filtrat das Lösungsmittel im Vakuum ab. Der Rückstand wird in Toluol aufgenommen, mit Wasser, verdünnter Natronlauge und nochmals mit Wasser gewaschen und über Natriumsulfat getrocknet. Das Lösungsmittel wird abdestilliert und der Rückstand umkristallisiert.

Nach dieser Verfahrensweise können z. B. die folgenden Produkte der Formel Ia hergestellt werden:

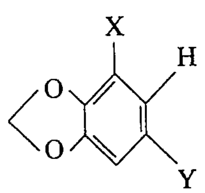

(Ia)

| Beispiel Nr. | X | Y | Ausbeute (% der Theorie) | Schmelzpunkt (°C) |
|---|---|---|---|---|
| 1 | Cl | Cl | 72 | 55 |
| 2 | Cl | Br | 40 | 68 |
| 3 | Br | Cl | 43 | 67 |
| 4 | Br | Br | 42 | 81 |

### b) Chlorierung von Benzodioxolen der Formel Ia

Zu 0,1 Mol 3,5-Dihalogen-1,2-methylendioxybenzol (Ia), gelöst in 100 ml Methylenchlorid, tropft man bei Raumtemperatur 0,11 Mol Sulfurylchlorid und erhitzt die Mischung 2 Stunden unter Rühren zum

Sieden. Nach dem Abkühlen wäscht man mit Wasser, Natriumbicarbonatlösung und nochmals mit Wasser, trocknet über Calciumchlorid, destilliert das Lösungsmittel im Vakuum ab und kristallisiert den Rückstand um.

c) Bromierung von Benzodioxolen der Formel Ia

Zu einer Lösung von 0,1 Mol 3,5-Dihalogen-1,2-methylendioxbenzol (Ia) in 150 ml Eisessig tropft man bei Raumtemperatur eine Lösung von 0,105 Mol Brom in 10 ml Eisessig und rührt die Mischung 4 Stunden bei Raumtemperatur. Anschließend wird die Reaktionsmischung in Wasser gegossen, mit Methylenchlorid extrahiert, die organische Phase mit Wasser gewaschen und über Calciumchlorid getrocknet. Nach Abdestillieren des Lösungsmittels im Vakuum wird der Rückstand umkristallisiert.

Nach (b) bzw. (c) können z. B. die folgenden 3,4,5-Trihalogen-1,2-methylendioxybenzodioxole der Formel Ib hergestellt werden:

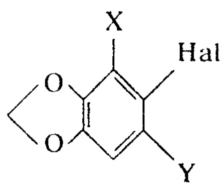

(Ib)

| Beispiel Nr. | X | Y | Hal | Ausbeute (% der Theorie) | Schmelzpunkt (°C) |
|---|---|---|---|---|---|
| 5 | Cl | Cl | Cl | 44 | 110 |
| 6 | Br | Br | Cl | 48 | 122 |
| 7 | Br | Cl | Cl | 35 | 105 |
| 8 | Cl | Cl | Br | 89 | 86 |

d) Chlormethylierung von Benzodioxolen der Formel Ia

0,5 Mol 3,5-Dihalogen-1,2-methylendioxybenzol (Ia) werden mit 70 ml Formalin in 300 ml konzentrierter Salzsäure bei 50—60° C 24 Stunden lang gerührt. Nach Abkühlen der Reaktionsmischung wird mit Toluol extrahiert, die organische Phase mit Wasser neutral gewaschen und über Natriumsulfat getrocknet. Das Lösungsmittel wird im Vakuum abgezogen und der Rückstand durch Vakuumdestillation gereinigt.

Beispiel 9

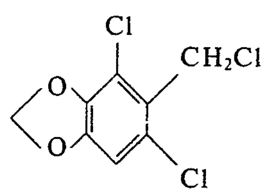

3,5-Dichlor-4-chlormethyl-1,2-methylendioxybenzol
mit dem Siedepunkt 140° C/267 Pa (2 Torr) und dem Schmelzpunkt 64° C in
74%iger Ausbeute der Theorie

e) Herstellung von Benzodioxolen der Formel Id

0,5 Mol 3,5-Dihalogen-4-chlormethyl-1,2-methylendioxybenzol (Ic) werden in 1000 ml Toluol gelöst, mit 1,0 Mol Triäthylamin versetzt und nach Zugabe von 10 g Raney-Nickel bei 80° C unter 65 bar Wasserstoff hydriert. Nach Abkühlen des Gemisches wird von Katalysator und ausgefallenem Triäthylammoniumchlorid abfiltriert, die organische Phase gewaschen und über Natriumsulfat

getrocknet. Das Lösungsmittel wird im Vakuum abgezogen und der Rückstand durch Vakuumdestillation gereinigt.

Beispiel 10

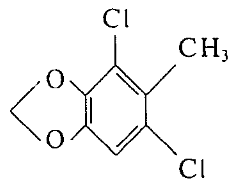

3,5-Dichlor-4-methyl-1,2-methylendioxybenzol mit dem Siedepunkt 98° C/267 Pa (2 Torr) und dem Schmelzpunkt 80° C in 35%iger Ausbeute der Theorie

f) Herstellung von Benzodioxolen der Formel Ie

0,5 Mol 3,5-Dihalogen-4-chlormethyl-methylendioxybenzol (Ic) werden in 500 ml Eisessig gelöst und mit 0,6 Mol wasserfreiem Natriumacetat versetzt. Unter Rühren wird die Mischung über Nacht unter Rückfluß zum Sieden erhitzt. Nach Abkühlen wird die Reaktionsmischung auf Eiswasser gegossen. Das dabei ausgefallene Produkt wird mit Wasser, dann mit Petroläther gewaschen und getrocknet.

Beispiel 11

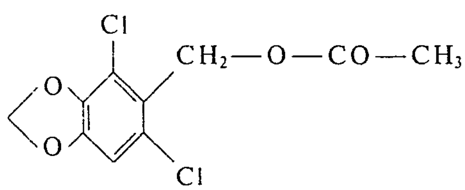

3,5-Dichlor-4-acetoxymethyl-1,2-methylendioxybenzol mit dem Schmelzpunkt 64° C in einer Ausbeute von 98% der Theorie

g) Herstellung von Benzodioxolen der Formel If

Zu einer Lösung von 1,05 Mol Natriumcyanid in 500 ml Dimethylformamid gibt man eine Lösung von 5 g Kaliumjodid in 20 ml Wasser und dosiert bei einer Innentemperatur von 60°C 1,0 Mol 3,5-Dihalogen-4-chlormethyl-1,2-methylendioxybenzol (Ic) so ein, daß die leicht exotherme Reaktion bei 60° C abläuft. Nach 5 Stunden Rühren bei Raumtemperatur gießt man die Mischung in 3 l Wasser, extrahiert mit Toluol, wäscht die organische Phase neutral, trocknet über Natriumsulfat, klärt die Lösung mit Aktivkohle/Tonsil und zieht das Lösungsmittel im Vakuum ab. Der kristalline Rückstand wird mit Äther/Petroläther (1 : 1) aufgeschlämmt, abgesaugt und getrocknet.

Beispiel 12

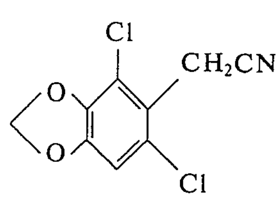

3,5-Dichlor-4-cyanomethyl-1,2-methylendioxybenzol mit dem Schmelzpunkt 115° C in einer Ausbeute von 65% der Theorie

h) Herstellung von Benzodioxolen der Formel I I

Zu einer Suspension von 1,1 Mol Kalium-tert.-butylat in 1,5 l Toluol gibt man bei 25 – 30° C unter leichtem Kühlen 1,0 Mol 3,5-Dihalogen-4-cyanomethyl-1,2-methylendioxybenzol (If), rührt die Mischung 30 Minuten bei 40 – 50° C und tropft dann bei 50° C 1,1 Mol Halogenalkan, -alken bzw. -alkin

zu. Nach 3 Stunden Rühren unter Rückfluß wird abgekühlt, mit Wasser versetzt, die organische Phase neutral gewaschen und über Natriumsulfat getrocknet. Das Lösungsmittel wird im Vakuum abgezogen und der Rückstand durch Destillation bzw. Umkristallisation gereinigt.

In analoger Weise können folgende Verbindungen hergestellt werden:

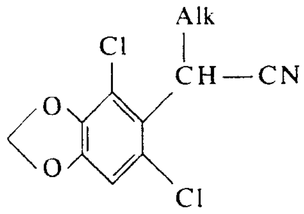

(II)

| Beispiel Nr. | Alk | Ausbeute (% der Theorie) | Schmelzpunkt (°C); Siedepunkt (°C/Pa (Torr)) | Chem.-Nr. |
|---|---|---|---|---|
| 13 | $CH_3$ | 70 | 103 | MVK 1371 |
| 14 | $C_2H_5$ | 73 | 160/267 (2) | MVK 1372 |
| 15 | $C_3H_7$-iso | 42 | 166/267 (2) | MVK 1374 |
| 16 | $CH_2{=}CH_2{-}CH_2{-}$ | 65 | 168/267 (2) | MVK 1373 |

Analog der Vorschrift (b) bzw. (c) wird erhalten

| Beispiel Nr. | X | Y | R |
|---|---|---|---|
| 17 | Cl | Br | Cl |

**Patentansprüche**

1. Benzodioxol-Derivate der Formel (I)

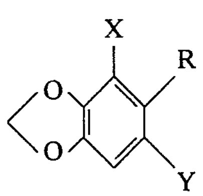

(I)

in welcher

R für Wasserstoff, Halogen, Methyl, Chlormethyl, Formyl, Acetoxymethyl und Cyano sowie für den Rest der Formel

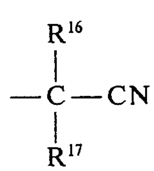

(VI)

worin

$R^{16}$ für Wasserstoff oder Methyl steht,
$R^{17}$ für Wasserstoff, geradkettiges oder verzweigtes Alkyl mit 1 bis 5 Kohlenstoffatomen, Allyl, Propargyl oder Benzyl steht sowie
X und Y für gleiches oder verschiedenes Halogen stehen.

2. Benzodioxol-Derivat der Formel

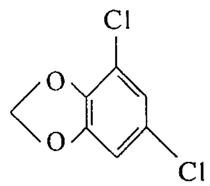

3. Verfahren zur Herstellung der Benzodioxolderivate der Formel (I) gemäß Anspruch 1, dadurch gekennzeichnet, daß man

a) 1,2-Dihydroxybenzole der Formel (VII)

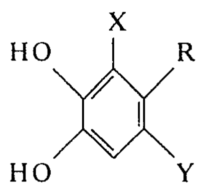

(VII)

worin

R, X und Y die oben angegebene Bedeutung haben,

mit Dihalogenmethanen der Formel (VIII)

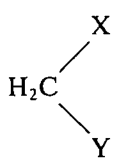

(VIII)

worin

X und Y die oben angegebene Bedeutung haben,

in Gegenwart eines Säureakzeptors und gegebenenfalls in Gegenwart eines Verdünnungsmittels umsetzt oder indem man

b) Benzodioxolderivate der Formel (Ia)

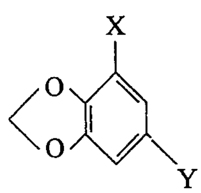

(Ia)

in welcher

X und Y die oben angegebene Bedeutung haben,

mit Halogenierungsmitteln oder mit Formaldehyd/HCl zu Verbindungen der Formel (I) umsetzt, in der X und Y die oben angegebene Bedeutung haben und R für Halogen oder $CH_2-Cl$ steht, oder indem man

c) Benzodioxolderivate der Formel (I c)

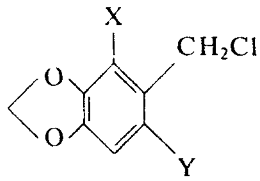

(Ic)

in welcher

X und Y die oben angegebene Bedeutung haben

durch katalytische Hydrierung in Verbindungen der Formel (I) umwandelt, in der R für Methyl steht bzw. durch Reaktion mit Verbindungen, welche das Acetat- bzw. Cyanidion enthalten, z. B. mit Natriumacetat bzw. -cyanid, in Verbindungen der Formel (I) umwandelt, in der R für Acetoxymethyl bzw. Cyanomethyl steht bzw. durch Umsetzung mit Hexamethylentetramin in Verbindungen der Formel (I) überführt, in der R für Formyl steht,

d) Benzodioxolderivate der Formel (I g)

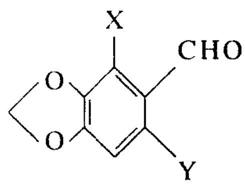

(Ig)

in welcher

X und Y die oben angegebene Bedeutung haben, mit Hydroxylamin in die entsprechenden Oxime überführt und daraus mit Dehydratisierungsmitteln die Verbindungen der Formel (I), in welcher R für Cyano steht, erzeugt,

e) Benzodioxolderivate der Formel (I f)

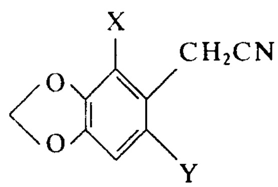

(If)

in welcher

X und Y die oben angegebene Bedeutung haben,

mit Halogenalkanen-alkenen oder -alkinen zu Verbindungen der Formel (II)

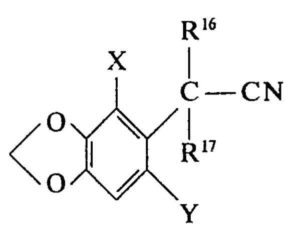

(I I)

in welcher

$R^{16}$ für Wasserstoff oder Methyl steht,
$R^{17}$ für Wasserstoff, geradkettiges oder verzweigtes Alkyl mit 1 bis 5, insbesondere 1 bis 3 Kohlenstoffatomen, Allyl, Propargyl oder Benzyl steht und
X und Y die angegebene Bedeutung haben,

umsetzt.

4. Insektizide und akarizide Mittel, gekennzeichnet durch einen Gehalt an einer Wirkstoffkombination, bestehend aus Benzodioxolderivaten der Formel I gemäß Anspruch 1 und

A) Carbamaten und/oder
B) Carbonsäureestern und/oder
C) Phosphorsäureestern und/oder
D) Halogenalkanen.

5. Insektizide und akarizide Mittel gemäß Anspruch 4, dadurch gekennzeichnet, daß in der Wirkstoffkombination das Gewichtsverhältnis von Benzodioxolderivaten zu Wirkstoffen zwischen 0,1 : 10 und 10 : 0,1 liegt.

6. Verwendung von Wirkstoffkombinationen gemäß Anspruch 4 oder 5 zur Bekämpfung von Insekten und Spinnentieren.

7. Verfahren zur Herstellung von insektiziden und akariziden Mitteln, dadurch gekennzeichnet, daß man eine Wirkstoffkombination gemäß Anspruch 4 oder 5 mit Streckmitteln und/oder oberflächenaktiven Mitteln vermischt.

**Claims**

1. Benzodioxole derivatives of the formula (I)

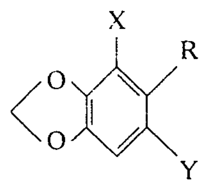

(I)

in which

R represents hydrogen, halogen, methyl, chloromethyl, formyl, acetoxymethyl and cyano and the radical of the formula

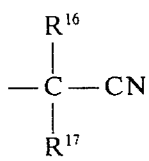

(VI)

wherein

$R^{16}$ represents hydrogen or methyl,
$R^{17}$ represents hydrogen, straight-chain or branched alkyl with 1 to 5 carbon atoms, allyl, propargyl or benzyl and
X and Y which may be identical or different, each represent halogen.

2. Benzodioxole derivative of the formula

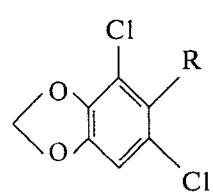

3. Process for the preparation of the benzodioxole derivatives of the formula (I) according to Claim 1, characterised in that

a) 1,2-dihydroxybenzenes of the formula (VII)

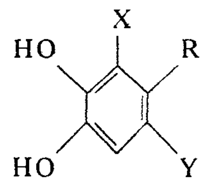

(VII)

wherein

R, X and Y have the meaning indicated above,

are reacted with dihalogenomethanes of the formula (VIII)

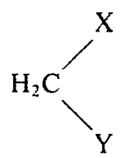

(VIII)

wherein

X and Y have the meaning indicated above,

in the presence of an acid acceptor and if appropriate in the presence of a diluent, or in that

b) benzodioxole derivatives of the formula (Ia)

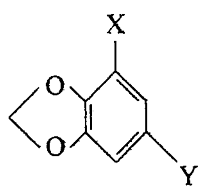

(Ia)

in which

X and Y have the meaning indicated above

are reacted with halogenation agents or with formaldehyde/HCl to give compounds of the formula (I) in which

X and Y have the meaning indicated above and
R represents halogen or $CH_2-Cl$,

or in that

c) benzodioxole derivatives of the formula (Ic)

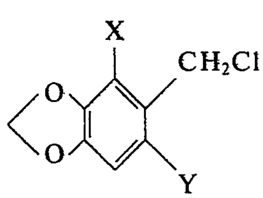

(Ic)

in which

X and Y have the meaning indicated above

are converted by catalytic hydrogenation into compounds of the formula (I) in which

R represents methyl

or are converted, by reaction with compounds which contain the acetate ion or cyanide ion, e.g. with sodium acetate or sodium cyanide, into compounds of the formula (I), in which

R represents acetoxymethyl or cyanomethyl

or are converted, by reaction with hexamethylenetetramine, into compounds of the formula (I), in which

R represents formyl,

d) benzodioxole derivatives of the formula (Ig)

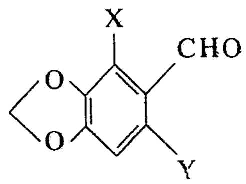

(Ig)

in which

X and Y have the meaning indicated above, are converted into the corresponding oximes by means of hydroxylamine and the oximes are treated with dehydrating agents to produce the compounds of the formula (I) in which
R represents cyano,

e) benzodioxole derivatives of the formula (If)

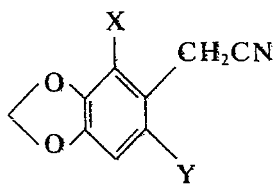

(If)

in which

X and Y have the meaning indicated above

are reacted with halogenoalkanes, halogenoalkenes or halogenoalkynes to give compounds of the formula (Il)

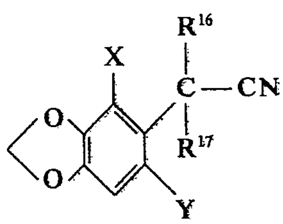

(Il)

in which

$R^{16}$ represents hydrogen or methyl,
$R^{17}$ represents hydrogen, straight-chain or branched alkyl with 1 to 5, in particular 1 to 3 carbon atoms, allyl, propargyl or benzyl and
X and Y have the meaning given above.

4. Insecticidal and acaricidal agents, characterised in that they contain a combination of active compounds consisting of benzodioxole derivatives of the formula I according to Claim 1 and

(A) carbamates and/or
(B) carboxylic acid esters and/or
(C) phosphoric acid esters and/or
(D) halogenoalkanes.

5. Insecticidal and acaricidal agents according to Claim 4, characterised in that in the combination of active compounds the weight ratio of benzodioxole derivatives to the active compounds is between 0.1 : 10 and 10 : 0.1.

6. Use of the combination of active compounds according to Claim 4 or 5 for combating insects and arachnidae.

7. Process for the preparation of insecticidal and acaricidal agents, characterised in that a combination of active compounds according to Claim 4 or 5 is mixed with extenders and/or surface-active agents.

**Revendications**

1. Dérivés benzodioxoliques de formule (I):

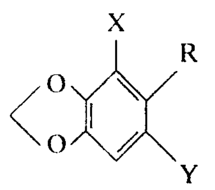

(I)

dans laquelle

R est l'hydrogène, un halogène, un reste méthyle, chlorométhyle, formyle, acétoxyméthyle et cyano ainsi que le reste de formule:

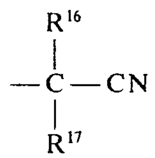

(VI)

dans laquelle

$R^{16}$ est l'hydrogène ou le reste méthyle,
$R^{17}$ est l'hydrogène, un reste alkyle à chaîne droite ou ramifié ayant I à 5 atomes de carbone, allyle, propargyle ou benzyle et
X and Y représentent le même halogène ou un halogène différent.

2. Dérivé benzodioxolique de formule:

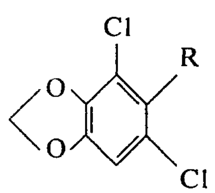

3. Procédé de production de dérivés benzodioxoliques de formule (I) suivant la revendication I, caractérisé en ce que:

a) on fait réagir des 1,2-dihydroxybenzènes de formule (VII):

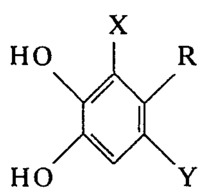

(VII)

dans laquelle

R, X et Y ont la définition donnée ci-dessus,

avec des dihalogénométhanes de formule (VIII):

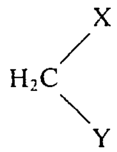

(VIII)

dans laquelle

X et Y ont la définition indiquée ci-dessus,

en présence d'un accepteur d'acide et le cas échéant en présence d'un diluant, ou bien

b) on fait réagir des dérivés benzodioxoliques de formule (I a):

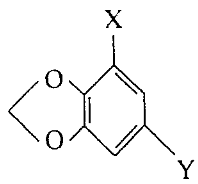

(I a)

dans laquelle

X et Y ont la définition indiquée ci-dessus

avec des agents d'halogénation ou avec le formaldéhyde/HCl pour former des composés de formule (I) dans laquelle X et Y ont la définition indiquée ci-dessus et R est un halogène ou un reste $CH_2-Cl$, ou bien

c) on transforme des dérivés benzodioxoliques de formule (Ic):

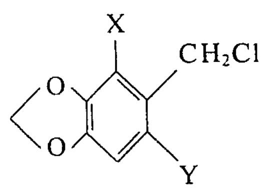

(I c)

dans laquelle

X et Y ont la définition indiquée ci-dessus

par hydrogénation catalytique en composés de formule (I) dans laquelle R est le reste méthyle ou on les transforme par réaction avec des composés qui renferment l'ion acétate ou cyanure, par exemple avec l'acétate de sodium ou le cyanure de sodium, en composés de formule (I) dans laquelle R est le reste acétoxyméthyle ou cyanométhyle, ou on les transforme par réaction avec l'hexaméthylènetétramine en composés de formule (I) dans laquelle R est le reste formyle,

d) on transforme des dérivés benzodioxoliques de formule (Ig):

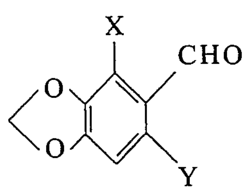

(Ig)

dans laquelle

X et Y ont la définition indiquée ci-dessus,

avec l'hydroxylamine en les oximes correspondantes à partir desquelles on produit avec des agents déshydratants les composés de formule I dans laquelle R est le reste cyano,

e) on fait réagir des dérivés benzodioxoliques de formule (If):

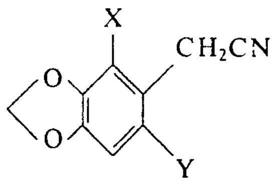

(If)

dans laquelle

X et Y ont la définition indiquée ci-dessus

avec des halogénalcanes-alcènes ou -alcynes pour former des composés de formule (II):

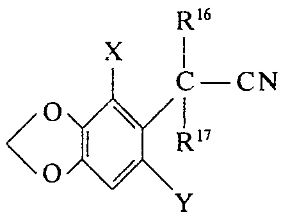

(II)

dans laquelle

$R^{16}$ est l'hydrogène ou le reste méthyle,
$R^{17}$ est l'hydrogène, un reste alkyle à chaîne droite ou ramifié ayant 1 à 5, notamment 1 à 3 atomes de carbone, le reste allyle, propargyle ou benzyle et
X et Y ont la définition indiquée ci-dessus.

4. Compositions insecticides et acaricides, caractérisées par une teneur en une association de substances actives, formée de dérivés benzodioxoliques de formule I suivant la revendication 1 et

A) de carbamates, et/ou
B) d'esters d'acides carboxyliques, et/ou
C) d'esters d'acide phosphorique, et/ou
D) d'halogénalcanes.

5. Compositions insecticides et acaricides suivant la revendication 4, caractérisées en ce que le rapport en poids des dérivés benzodioxoliques aux substances actives dans l'association de substances actives se situe entre 0,1 : 10 et 10 : 0,1.

6. Utilisation de l'association de substances actives suivant la revendication 4 ou 5 pour la lutte contre des insectes et des acariens.

7. Procédé de préparation de compositions insecticides et acaricides, caractérisé en ce qu'on mélange une association de substances actives suivant la revendication 4 ou 5 avec des diluants et/ou des agents tensio-actifs.